(19) Europäisches Patentamt
European Patent Office
Office européen des brevets

(11) **EP 4 467 529 A1**

(12) # EUROPEAN PATENT APPLICATION
published in accordance with Art. 153(4) EPC

(43) Date of publication:
**27.11.2024 Bulletin 2024/48**

(21) Application number: **23756506.4**

(22) Date of filing: **21.02.2023**

(51) International Patent Classification (IPC):
***C07C 17/389*** *(2006.01)* ***B01D 53/04*** *(2006.01)*
***B01J 20/26*** *(2006.01)* ***B01J 20/28*** *(2006.01)*
***C07C 19/08*** *(2006.01)* ***C07C 19/10*** *(2006.01)*
***C07C 55/06*** *(2006.01)* ***C07F 1/08*** *(2006.01)*
***C07F 3/06*** *(2006.01)* ***C07F 5/06*** *(2006.01)*

(52) Cooperative Patent Classification (CPC):
(C-Sets available)
**C07C 17/389; B01D 53/02; B01D 53/04; B01J 20/22; B01J 20/26; B01J 20/28; C07F 1/08; C07F 3/06; C07F 5/06** (Cont.)

(86) International application number:
**PCT/JP2023/006287**

(87) International publication number:
**WO 2023/157982 (24.08.2023 Gazette 2023/34)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.02.2022 JP 2022024980**
**28.09.2022 JP 2022155183**

(71) Applicants:
- **DAIKIN INDUSTRIES, LTD.**
  **Osaka-shi, Osaka 530-0001 (JP)**
- **Atomis Inc.**
  **Kobe-shi, Hyogo 650-0047 (JP)**

(72) Inventors:
- **KAGAWA, Michiru**
  **Osaka-Shi, Osaka 530-0001 (JP)**
- **TANAKA, Yoshinori**
  **Osaka-Shi, Osaka 530-0001 (JP)**
- **SUMIDA, Kenji**
  **Kobe-shi, Hyogo 650-0047 (JP)**
- **FUJII, Yusuke**
  **Kobe-shi, Hyogo 650-0047 (JP)**

(74) Representative: **Hoffmann Eitle**
**Patent- und Rechtsanwälte PartmbB**
**Arabellastraße 30**
**81925 München (DE)**

(54) **ISOLATION METHOD**

(57) A method for separating a fluorocarbon, comprising:
separating a fluorocarbon having n carbon atoms from a mixture comprising the fluorocarbon having n carbon atoms and a fluorocarbon having n + m or more carbon atoms,
wherein the fluorocarbon having n carbon atoms is separated by bringing the mixture and an adsorbent into contact with each other, and
wherein n is an integer of 1 or more, and m is an integer of 1 or more.

EP 4 467 529 A1

(52) Cooperative Patent Classification (CPC): (Cont.)

C-Sets
**C07C 17/389, C07C 19/08;**
**C07C 17/389, C07C 19/10**

## Description

Technical Field

The present disclosure relates to a separation method.

Background Art

A known method for separating fluorocarbon includes distilling a mixture of fluorocarbons. Patent Literature 1 describes obtaining a mixture for extraction from a mixture containing 2,3,3,3-tetrafluoropropene and hexafluoropropene; and an extraction solvent, and distilling the mixture for extraction to obtain a distillate containing hexafluoropropene as a main component, and a bottom containing 2,3,3,3 -tetrafluoropropene.

Citation List

Patent Literature

Patent Literature 1: JP2018-002602A

Summary

Technical Problem

In a distillation method as described in Patent Literature 1, it has been found that the separation of compounds having similar boiling points or azeotropic compounds is difficult, and that disadvantageously, separation of a fluorocarbon, for example, a fluorocarbon having one carbon atom, may be achieved insufficiently.

The present disclosure is to provide a method for separating a fluorocarbon having a specific number of carbon atoms and is to provide, for example, a method for separating a fluorocarbon having one carbon atom.

Solution to Problem

The present disclosure comprises the following aspects.

A method for separating a fluorocarbon, comprising:

separating a fluorocarbon having n carbon atoms from a mixture comprising the fluorocarbon having n carbon atoms and a fluorocarbon having n + m or more carbon atoms,
wherein the fluorocarbon having n carbon atoms is separated by bringing the mixture and an adsorbent into contact with each other, and
n is an integer of 1 or higher, and m is an integer of 1 or more.

[2] The method according to [1], wherein the mixture and the adsorbent are brought into contact with each other at a temperature of -10°C or higher and 30°C or lower.
[3] The method according to [1] or [2], wherein n is an integer of 1 or more and 2 or less.
[4] The method according to any one of [1] to [3], wherein m is an integer of 1 or more and 3 or less.
[5] The method according to any one of [1] to [4], wherein the fluorocarbon having the number n of carbon atoms is at least one selected from the group consisting of difluoromethane and difluorochloromethane.
[6] The method according to any one of [1] to [5], wherein the fluorocarbon having the number n + m or more of carbon atoms includes a fluorocarbon having 2 or 3 carbon atoms.
[7] The method according to any one of [1] to [6], wherein the adsorbent contains a porous body, and the porous body has an effective pore diameter of 0.46 nm or less.
[8] The method according to [7], wherein the porous body has a metal-organic framework.
[9] The method according to [8], wherein the metal-organic framework has a metal ion and one or more organic ligands, and
the organic ligand has two or more groups capable of forming a coordinate bond to the metal ion, per molecule.
[10] The method according to [8], wherein the metal-organic framework has a metal ion.
[11] The method according to [8], wherein the metal-organic framework has one or more organic ligands, and
the organic ligand has two or more groups capable of forming a coordinate bond to the metal ion, per molecule.
[12] The method according to [9], wherein the metal ion comprises zinc, and the organic ligands include oxalic acid and

1,2,4-triazole.

[13] The method according to any one of [7] to [12], wherein the adsorbent further comprises a resin.

[14] The isolation method according to any one of [1] to [13], wherein the adsorbent is in a form of powder, granules, flakes, or pellets.

[15] The isolation method according to any one of [1] to [14], wherein the mixture is an azeotropic mixture or pseudoazeotropic mixture of fluorocarbons.

[16] The isolation method according to any one of [1] to [15], further comprising: purifying the isolated fluorocarbon having the number n of carbon atoms.

[17] A composite material comprising: a metal-organic framework; and a fluorocarbon having the number n of carbon atoms,

wherein the metal-organic framework has a metal ion and one or more organic ligands,
the metal ion includes zinc, the organic ligands include oxalic acid and 1,2,4-triazole, and
n is an integer of 1 or more.

Advantageous Effects

According to the present disclosure, a fluorocarbon having a specific number of carbon atoms, for example, a fluorocarbon having one carbon atom, can be separated from a mixture comprising two or more fluorocarbons.

Brief Description of the Drawings

Figure 1 shows adsorption isotherms in Example 1.
Figure 2 shows adsorption isotherms in Example 1.
Figure 3 shows adsorption isotherms in Comparative Examples 2 and 3.
Figure 4 shows adsorption breakthrough curves in Example 1.
Figure 5 shows adsorption breakthrough curves in Example 2.
Figure 6 shows adsorption isotherms in repeated adsorption and desorption in Example 1.
Figure 7 shows adsorption isotherms after high temperature and high humidity storage, in Example 1.

Description of Embodiments

A separation method of the present disclosure may comprise separating a specific fluorocarbon from a mixture comprising two or more fluorocarbons, and the specific fluorocarbon can be separated by bringing the mixture and an adsorbent into contact with each other.

A separation method in one embodiment of the present disclosure comprises;

separating a fluorocarbon having n carbon atoms from a mixture comprising the fluorocarbon having n carbon atoms and a fluorocarbon having n + m or more carbon atoms, and
the fluorocarbon having n carbon atoms is separated by bringing the mixture and an adsorbent into contact with each other.

In the separation method of the present disclosure, the fluorocarbon having n carbon atoms is adsorbed to the adsorbent, whereby the fluorocarbon having n carbon atoms can be separated from the mixture. In one embodiment, the mixture in a gas state is brought into contact with such an adsorbent.

In one embodiment, such a separation method may comprise;

separating a fluorocarbon having one carbon atom from a mixture comprising the fluorocarbon having one carbon atom and a fluorocarbon having two or more carbon atoms, and
the fluorocarbon having one carbon atom is separated by bringing the mixture and an adsorbent into contact with each other.

In another embodiment, such a separation method may comprise;

separating a fluorocarbon having 2 carbon atoms from a mixture comprising the fluorocarbon having 2 carbon atoms and a fluorocarbon having 3 or more carbon atoms, and

the fluorocarbon having 2 carbon atoms is separated by bringing the mixture and an adsorbent into contact with each other.

In still another embodiment, such a separation method may comprise;

separating a fluorocarbon having one carbon atom from a mixture comprising the fluorocarbon having one carbon atom and a fluorocarbon having 3 or more carbon atoms, and
the fluorocarbon having one carbon atom is separated by bringing the mixture and an adsorbent into contact with each other.

(Mixture)

The mixture comprises a fluorocarbon having n carbon atoms. n is an integer of 1 or more and preferably 1 or more and 2 or less.
In one embodiment, the molecular diameter of the fluorocarbon having n carbon atoms is, for example, 0.30 nm or more and 0.430 nm or less and preferably 0.320 nm or more and 0.420 nm or less. The molecular diameter of the fluorocarbon having n carbon atoms is, for example, 0.300 nm or more and preferably 0.320 nm or more and is, for example, 0.430 nm or less and preferably 0.420 nm or less.
In another embodiment, the molecular diameter of the fluorocarbon having n carbon atoms is, for example, 0.300 nm or more and 0.460 nm or less and preferably 0.320 nm or more and 0.440 nm or less. The molecular diameter of the fluorocarbon having n carbon atoms is, for example, 0.300 nm or more and preferably 0.320 nm or more and is, for example, 0.460 nm or less and preferably 0.440 nm or less.
In still another embodiment, the molecular diameter of the fluorocarbon having n carbon atoms is, for example, 0.480 nm or more and 1.0 nm or less and preferably 0.490 nm or more and 0.980 nm or less. In addition, the molecular diameter of the fluorocarbon having n carbon atoms is, for example, 0.480 nm or more and preferably 0.490 nm or more and is, for example, 1.0 nm or less and preferably 0.800 nm or less.
The molecular diameter is a value that is calculated by the following formula:

$$d = (ma/(3 \times 2^{1/2}\eta\pi))^{1/2} \quad \text{... (x1)}$$

wherein d represents the molecular diameter, m represents the mass of one molecule, a represents the molecular speed, and $\eta$ represents the viscosity coefficient.
a is calculated by the following formula:

$$a = (\gamma RT/M)^{1/2} \quad \text{... (x2)}$$

wherein $\gamma$ is the specific heat ratio and approximates 1.333, R is the gas constant, T is the absolute temperature, and M is the molar mass of the molecule.
$\eta$ may be measured by, for example, a capillary method.

The boiling point of the fluorocarbon having n carbon atoms may be, for example, -80°C or higher and -20°C or lower and, furthermore, may be -60°C or higher and -30°C or lower. In one embodiment, the fluorocarbon having n carbon atoms is present in the form of a gas in the mixture.
Examples of the fluorocarbon having n carbon atoms comprise hydrochlorofluorocarbon and hydrofluorocarbon. Examples of the hydrochlorofluorocarbon comprise chlorodifluoromethane, and examples of the hydrofluorocarbon comprise difluoromethane. In the present disclosure, the fluorocarbon having n carbon atoms is also referred to as an adsorbate.
The fluorocarbon having n carbon atoms may comprise one or more compounds. When the fluorocarbon having n carbon atoms comprises two or more compounds, each compound preferably has a molecular diameter and a boiling point within the respective ranges described above.
The content of the fluorocarbon having n carbon atoms in the mixture may be, for example, 0.1 mol% or more and 50 mol% or less and, furthermore, may be 0.5 mol% or more and 20 mol% or less, based on the entire mixture.
The mixture comprises a fluorocarbon having n + m carbon atoms. m is an integer of 1 or more, preferably 1 or more and 3 or less and more preferably 1 or more and 2 or less.
The content of a fluorocarbon having 2 or 3 carbon atoms in the fluorocarbon having n + m or more carbon atoms may be, for example, 50 mol% or more and 100 mol% or less and may be 80 mol% or more and 100 mol% or less.
In one embodiment, the molecular diameter of the fluorocarbon having n + m or more carbon atoms is, for

example, more than 0.430 nm, may be 0.440 nm or more and 2.00 nm or less or may be 0.440 nm or more and 1.00 nm or less. The molecular diameter of the fluorocarbon having n + m or more carbon atoms is, for example, more than 0.430 nm and preferably 0.440 nm or more and is, for example, 2.00 nm or less and preferably 1.00 nm or less.

**In** another embodiment, the molecular diameter of the fluorocarbon having n + m or more carbon atoms is, for example, more than 0.460 nm, may be 0.470 nm or more and 2.00 nm or less or may be 0.470 nm or more and 1.00 nm or less. The molecular diameter of the fluorocarbon having n + m or more carbon atoms is, for example, more than 0.460 nm and preferably 0.470 nm or more and is, for example, 2.00 nm or less and preferably 1.00 nm or less.

In still another embodiment, the molecular diameter of the fluorocarbon having n + m or more carbon atoms is, for example, more than 1.0 nm, may be 1.10 nm or more and 2.00 nm or less or may be 1.10 nm or more and 1.50 nm or less. The molecular diameter of the fluorocarbon having n + m or more carbon atoms is, for example, more than 1.0 nm and preferably 1.10 nm or more and is, for example, 2.00 nm or less and preferably 1.50 nm or less.

The boiling point of the fluorocarbon having n + m or more carbon atoms may be, for example, -80°C or higher and -20°C or lower, and furthermore, may be - 60°C or higher and -30°C or lower and, particularly, -50°C or higher and -30°C or lower. In one embodiment, the fluorocarbon having n + m or more carbon atoms is present in the form of a gas in the mixture.

The difference between the boiling point of the fluorocarbon having n carbon atoms and the boiling point of the fluorocarbon having n + m or more carbon atoms may be, for example, 0°C or more and 20°C or less and, furthermore, may be 0°C or more and 10°C or less. Even when the boiling point of the fluorocarbon having n carbon atoms and the boiling point of the fluorocarbon having n + m or more carbon atoms are close to each other, the fluorocarbon having n carbon atoms can be separated by the separation method of the present disclosure.

The fluorocarbon having n + m or more carbon atoms may be a saturated fluorocarbon or an unsaturated fluorocarbon and is preferably a saturated fluorocarbon.

Examples of the fluorocarbon having n + m or more carbon atoms comprise hydrochlorofluorocarbons and hydrofluorocarbons. Examples of the hydrochlorofluorocarbons comprise hydrochlorofluorocarbon having two carbon atoms such as chlorotrifluoroethane. Examples of the hydrofluorocarbons comprise difluoroethane (for example, 1,1-difluoroethane and 1,2-difluoroethane, particularly, 1,1-difluoroethane), trifluoroethane (for example, 1,1,1-trifluoroethane and 1,1,2-trifluoroethane, particularly 1,1,1-trifluoroethane), tetrafluoroethane (for example, 1,1,1,2-tetrafluoroethane and 1, 1,2,2-tetrafluoroethane, particularly, 1,1,1,2-tetrafluoroethane); hydrofluorocarbons having 2 carbon atoms such as pentafluoroethane; and hydrofluorocarbons having 3 carbon atoms such as tetrafluoropropene and hexafluoropropene. One or more of these may be comprised in the fluorocarbons having n + m or more carbon atoms.

In a preferable embodiment, the fluorocarbon having n + m or more carbon atoms may be, for example, a fluorocarbon having 2 to 5 carbon atoms, and may preferably be a fluorocarbon having 2 or 3 carbon atoms.

The fluorocarbon having n + m or more carbon atoms may comprise one or more compounds. When the fluorocarbon having n + m or more carbon atoms comprises two or more compounds, each compound preferably has a molecular diameter and a boiling point within the respective ranges described above.

The contents of the fluorocarbon having n carbon atoms and the fluorocarbon having n + m or more carbon atoms in the mixture is preferably 90 mol% or more and 100 mol% or less and more preferably 95 mol% or more and 100 mol% or less, based on the entire mixture.

The mixture may be an azeotropic mixture or pseudoazeotropic mixture of fluorocarbons. According to the separation method of the present disclosure, a fluorocarbon having a specific number of carbon atoms can be separated even when the mixture is an azeotropic mixture or a pseudoazeotropic mixture. Examples of such an azeotropic mixture or pseudoazeotropic mixture comprise mixtures in which the difference between the boiling point of a fluorocarbon that is a separation target (in the present embodiment, the fluorocarbon having n carbon atoms) and the boiling point of a different fluorocarbon (in the present embodiment, the fluorocarbon having n + m or more carbon atoms) is 1°C or more and 20°C or less, furthermore, 3°C or more and 18°C or less.

In one embodiment, the fluorocarbon having n carbon atoms can be a fluorocarbon having 1 carbon atom, and the fluorocarbon having n + m or more carbon atoms can be a fluorocarbon having 2 or more carbon atoms.

In another embodiment, the fluorocarbon having n carbon atoms can be a fluorocarbon having 2 carbon atoms, and the fluorocarbon having n + m or more carbon atoms can be a fluorocarbon having 3 or more carbon atoms.

In still another embodiment, the fluorocarbon having n carbon atoms can be a fluorocarbon having 1 carbon atom, and the fluorocarbon having n + m or more carbon atoms can be a fluorocarbon having 3 or more carbon atoms.

In far still another embodiment, the fluorocarbon having n carbon atoms can be a fluorocarbon having 1 carbon atom, and the fluorocarbon having n + m or more carbon atoms can be a fluorocarbon having 4 or more carbon atoms.

In far still another embodiment, the fluorocarbon having n carbon atoms can be a fluorocarbon having 2 carbon atoms, and the fluorocarbon having n + m or more carbon atoms can be a fluorocarbon having 5 or more carbon atoms.

The mixture may comprise other compounds, in addition to the fluorocarbon having n carbon atoms and the fluorocarbon having n + m or more carbon atoms. The boiling point of such other compounds may be, for example, - 100°C or lower or -150°C or lower. In one embodiment, such other compounds are comprised in the mixture as a gas.

Examples of such other compounds comprise nitrogen, oxygen, carbon dioxide, and water.

(Adsorbent)

The adsorbent is capable of adsorbing the fluorocarbon having n carbon atoms. Typically, the adsorbent adsorbs the fluorocarbon having n carbon atoms, but does not adsorb the fluorocarbon having n + m or more carbon atoms. Therefore, when a mixture comprising the fluorocarbon having n carbon atoms and the fluorocarbon having n + m or more carbon atoms and the adsorbent are brought into contact with each other, it is possible to separate the fluorocarbon having n carbon atoms from the mixture.

The adsorbent preferably comprises a porous body having a pore, as an adsorptive medium. When the fluorocarbon having n carbon atoms is adsorbed onto such a pore, it is possible to separate the fluorocarbon having n carbon atoms from the mixture.

The effective pore diameter of the porous body is preferably smaller than the molecular diameter of the fluorocarbon having n + m or more carbon atoms. When the effective pore diameter is smaller than the molecular diameter of the fluorocarbon having n + m or more carbon atoms, it is possible to more reliably separate the fluorocarbon having n carbon atoms.

Here, the effective pore diameter is the pore diameter of the porous body that is determined from the degree of adsorption and the molecular diameter of the adsorbate when the porous body and the adsorbate are brought into contact with each other and, specifically, can be obtained by converting the relative pressure of a nitrogen adsorption isotherm into the effective pore diameter using a Horvath-Kawazoe method. As a conversion formula, a calculation formula described in J. Chem. Eng, Jpn., 1983, 16, 6, 470 to 475 may be used, and as a parameter value in the conversion formula, a value based on a combination of carbon and nitrogen may be used.

The effective pore diameter of the porous body is, for example, 0.30 nm or more and 0.46 nm or less and preferably 0.32 nm or more and 0.44 nm or less. In one embodiment, the effective pore diameter of the porous body is, for example, 0.30 nm or more and preferably 0.32 nm or more and is, for example, 0.46 nm or less and preferably 0.44 nm or less.

The specific surface area of the porous body can be, for example, 40 $m^2$/g or more, preferably 100 $m^2$/g or more and more preferably 300 $m^2$/g or more and may be, for example, 2,000 $m^2$/g or less and may be 1,000 $m^2$/g or less. The specific surface area of the porous body is calculated from the nitrogen gas adsorption isotherm using a BET method.

The equilibrium adsorption amount of the fluorocarbon having n carbon atoms to the porous body may be, for example, 1 g/10 g or more or 100 g/10 g or less as measured at a temperature of 303 K and a pressure within a range of 0.4 Pa to 1 MPa.

The porous body may be in the form of powder (particles), a film, granules, or a molded product (for example, pellets), for example, and is preferably a molded product and more preferably in the form of pellets. A pellet may have a cylindrical shape or a spherical shape. In the case of the cylindrical shape, the radius of the cylinder is preferably 1.6 mm or more and 6 mm or less, and the thickness of the cylinder is preferably 2 mm or more and 6 mm or less. In the case of the spherical shape, the spherical shape does not need to be truly spherical at all times, and the radius of the sphere is preferably 4 mm or more and 12 mm or less.

The porous body preferably comprises, for example, at least one selected from the group consisting of zeolite, activated carbon, silica gel and a metal-organic framework (MOF, or PCP: porous coordination polymer), and typically comprises a metal-organic framework.

The metal-organic framework preferably comprises metal ions and one or more kinds of organic ligands. Typically, the organic ligands preferably comprise two or more groups capable of forming a coordinate bond to the metal ion, per molecule. When the organic ligands coordinates to the metal ions, a highly regular and three-dimensionally continuous structure (for example, a network structure) is formed, and pores are formed between the metal ions and the organic ligands. The combination of the kinds of the metal ions and the organic ligands could control the effective pore diameter or flexibility of the metal-organic framework and the interaction between the metal-organic framework and the adsorbate in such a three-dimensional structure.

In addition, the metal-organic framework exhibits an adsorption capacity (maximum adsorption capacity) that does not decrease even after the repeated adsorption and desorption of the adsorbate and thus exhibits high durability to repetition. Furthermore, the metal-organic framework is less likely to be affected by temperatures (heat) or humidity, exhibits an adsorption capacity that does not decrease even through storage at a high temperature and a high humidity for a long period of time and thus exhibits high durability to high-temperatures and high-humidities.

The metal ion is not limited, and examples thereof comprise the ions of metals selected from the group consisting of Group Ia, Group IIa, Group IIIa, Groups IVa to VIII and Groups Ib to VIb. Such metals can be preferably one or more selected from the group consisting of Mg, Ca, Sr, Ba, Sc, Y, Ti, Zr, Hf, V, Nb, Ta, Cr, Mo, W, Mn, Re, Fe, Ro, Os, Co, Rh, Ir, Ni, Pd, Pt, Cu, Ag, Au, Zn, Cd, Hg, Al, Ga, In, Tl, Si, Ge, Sn, Pb, As, Sb, Bi, La, Ce, Pr, Nd, Pm, Sm, En, Gd, Tb, Dy, Ho, Er, Tm or Yb, and can be more preferably one or more selected from the group consisting of Zn, Cu, Ti, Co, Mn, Al, Ca, Zr or Mg.

Specifically, such metal ions can be one or more selected from the group consisting of $Mg^{2+}$, $Ca^{2+}$, $Sr^{2+}$, $Ba^{2+}$, $Sc^{3+}$, $Y^{3+}$, $Ln^{3+}$, $Ti^{4+}$, $Zr^{4+}$, $Hf^{4+}$, $V^{4+}$, $V^{3+}$, $V^{2+}$, $Nb^{3+}$, $Ta^{3+}$ , $Cr^{3+}$, $Mo^{3+}$, $W^{3+}$, $Mn^{3+}$, $Mn^{2+}$, $Re^{3+}$, $Re^{2+}$, $Fe^{3+}$, $Fe^{2+}$, $Ru^{3+}$, $Ru^{2+}$, $Os^{3+}$, $Os^{2+}$, $Co^{3+}$, $Co^{2+}$, $Rh^{2+}$, $Rh^{+}$, $Ir^{24}$, $Ir^{+}$, $Ni^{2+}$, $Ni^{+}$, $Pd^{2+}$, $Pd^{+}$, $Pt^{2+}$, $Pt^{+}$, $Cu^{2+}$, $Cu^{+}$, $Ag^{+}$, $Au^{+}$, $Zn^{2+}$, $Cd^{2+}$, $Hg^{2+}$, $Al^{3+}$, $Ga^{3+}$, $In^{3+}$,$Tl^{3+}$, $Si^{4+}$, $Si^{2+}$, $Ge^{4+}$, $Ge^{2+}$, $Sn^{4+}$, $Sn^{2+}$, $Pb^{4+}$, $Pb^{2+}$, $As^{5+}$, $As^{3+}$, $As^{+}$, $Sb^{5+}$, $Sb^{3+}$, $Sb^{+}$, $Bi^{5+}$, $Bi^{3+}$, $Bi^{+}$, $La^{3+}$ $Ce^{3+}$, $Pr^{3+}$, $Nd^{3+}$, $Pm^{3+}$, $Sm^{3+}$, $En^{3+}$, $Gd^{3+}$, $Tb^{3+}$, $Dy^{3+}$, $Ho^{3+}$, $Er^{3+}$, $Tm^{3+}$ or $Yb^{3+}$ and can be more preferably one or more selected from the group consisting of $Zn^{2+}$, $Cu^{2+}$, $Cu^{+}$, $Ti^{4+}$, $Ti^{3+}$, $Co^{3+}$, $Co^{2+}$, $Mn^{3+}$, $Mn^{2+}$, $Al^{3+}$, $Ca^{2+}$, $Zr^{4+}$ or $Mg^{2+}$.

The number of types of the metal ions may be only 1 or 2 or more. The number of types of the metal ions is preferably 1.

In a preferable embodiment, the metal ion is a Zn ion and preferably $Zn^{2+}$.

The organic ligand is not limited, as long as the organic ligand has two or more groups capable of forming a coordinate bond to the metal ion, per molecule. The coordinate bond is formed of, for example, a functional group capable of forming a coordinate bond to the metal ion.

Examples of the functional group capable of forming the coordinate bond comprise -COOH, $-CS_2H$, $-NO_2$, $-B(OH)_2$, $-SO_3H$, $-Si(OH)_3$, $-Ge(OH)_3$, $-Sn(OH)_3$, - $Si(SH)_4$, $-Ge(SH)_4$, $-Sn(SH)_3$, $-PO_3H$, $-AsO_3H$, $-AsO_4H$, $-P(SH)_3$, $-As(SH)_3$, - $CH(RSH)_2$, $-C(RSH)_3$, $-CH(RNH_2)_2$, $-C(RNH_2)_3$, $-CH(ROH)_2$, $-C(ROH)_3$, - $CH(RCN)_3$ and $-C(RCN)_3$. In the formulae, R is a single bond, an alkylene group having 1 to 5 carbon atoms (for example, methylene, ethylene, n-propylene, i-propylene, n-butylene, i-butylene, tert-butylene or n-pentylene group), a divalent aromatic group having 6 to 14 carbon atoms (for example, phenylene) or a combination of the alkylene group and the aromatic group (for example, -phenylene-alkylene-phenylene). Alternatively, the functional group capable of forming a coordinate bond may be a hetero atom (for example, N, O, S, B, P, Si or Al) comprised in a heterocycle and may be preferably a nitrogen atom in a heterocycle.

In a preferable embodiment, the functional group capable of forming a coordinate bond can be -COOH, or a nitrogen atom in a heterocycle, for example.

The organic ligand preferably comprises the functional group so as to be a bi- or higher-dentate ligand. In such an organic ligand, the moiety other than the functional group is not limited as long as the organic ligand is capable of forming a coordinate bond to the metal ion.

In one embodiment, the organic ligand derives from a saturated or unsaturated aliphatic compound or aromatic compound. The ligand may be derived from a compound formed by bonding such an aliphatic compound to an aromatic compound bond (hereinafter, also referred to as "aliphatic aromatic compound").

An aliphatic moiety of the aliphatic compound or the aliphatic aromatic compound may be linear, branched or cyclic. When the aliphatic moiety is cyclic, the aliphatic moiety may comprise a plurality of rings. The aliphatic moiety of the aliphatic compound or the aliphatic aromatic compound preferably has 1 to 15 carbon atoms and more preferably has 1 to 10 carbon atoms, for example, 1, 2, 3, 4, 5, 6, 7, 8, 9 or 10 carbon atoms. In a preferable embodiment, the aliphatic moiety derives from methane, adamantane, acetylene, ethylene or butadiene.

An aromatic moiety of the aromatic compound or the aliphatic aromatic compound may have 1 or more rings, for example, 2, 3, 4 or 5 rings. These rings may or may not be condensed. The aromatic moiety of the aromatic compound or the aliphatic aromatic compound preferably has 1, 2 or 3 rings and more preferably 1 or 2 rings. Each ring of the compound may comprise at least one heteroatom, for example, N, O, S, B, P, Si or Al, preferably N, O or S, in the ring. The aromatic moiety of the aromatic compound or the aromatic aliphatic compound preferably has 1 or 2 rings having 6 carbon atoms. When there are 2 aromatic moieties, such two rings may or may not be condensed. In a preferable embodiment, such an aromatic moiety derives from benzene, naphthalene, biphenyl, bipyridyl or pyridyl.

In one embodiment, the organic ligand derives from dicarboxylic acid, tricarboxylic acid or tetracarboxylic acid.

Examples of the dicarboxylic acid comprise oxalic acid, succinic acid, tartaric acid, maleic acid, 1,4-butanedicarboxylic acid, 1,4-butenedicarboxylic acid, 4-oxopyran-2,6-dicarboxylic acid, 1,6-hexanedicarboxylic acid, decanedicarboxylic acid, 1,8-heptadecanedicarboxylic acid, 1,9-heptadecanedicarboxylic acid, heptadecanedicarboxylic acid, acetylenedicarboxylic acid, 1,2-benzenedicarboxylic acid, 1,3-benzenedicarboxylic acid, 2,3-pyridinedicarboxylic acid, pyridine-2,3-dicarboxylic acid, 1,3-butadiene-1,4-dicarboxylic acid, 1,4-benzenedicarboxylic acid, p-benzenedicarboxylic acid, imidazole-2,4-dicarboxylic acid, 2-methylquinoline-3,4-dicarboxylic acid, quinoline-2,4-dicarboxylic acid, quinoxaline-2,3-dicarboxylic acid, 6-chloroquinoxaline-2,3-dicarboxylic acid, 4,4'-diaminophenylmethane-3,3'-dicarboxylic acid, quinoline-3,4-dicarboxylic acid, 7-chloro-4-hydroxyquinoline-2,8-dicarboxylic acid, diimidodicarboxylic acid, pyridine-2,6-dicarboxylic acid, 2-methylimidazole-4,5-dicarboxylic acid, thiophene-3,4-dicarboxylic acid, 2-isopropylimidazole-4,5-dicarboxylic acid, tetrahydropyran-4,4-dicarboxylic acid, perylene-3,9-dicarboxylic acid, perylene dicarboxylic acid, PLURIOL E200-dicarboxylic acid, 3,6-dioxaoctanedicarboxylic acid, 3,5-cyclohexadiene-1,2-dicarboxylic acid, octanedicarboxylic acid, pentane-3,3-dicarboxylic acid, 4,4'-diamino-1,1'-biphenyl-3,3'-dicarboxylic acid, 4,4'-diaminobiphenyl-3,3'-dicarboxylic acid, benzidine-3,3'-dicarboxylic acid, 1,4-bis(phenylamino)benzene-2,5-dicarboxylic acid, 1,1'-binaphthyldicarboxylic acid, 7-chloro-8-methylquinoline-2,3-dicarboxylic acid, 1-anilino-anthraquinone-2,4'-dicarboxylic acid, polytetrahydrofuran 250-dicarboxylic acid, 1,4-bis(carboxymethyl)piperazine-2,3-dicarboxylic acid, 7-chloroquinoline-3,8-dicarboxylic acid, 1-(4-carboxy)phenyl-3-(4-chloro)phenylpyrazoline-4,5-dicarboxylic acid,

1,4,5,6,7,7-hexachloro-5-norbomene-2,3-dicarboxylic acid, phenylindanedicarboxylic acid, 1,3-dibenzyl-2-oxoimidazolidine-4,5-dicarboxylic acid, 1,4-cyclohexanedicarboxylic acid, naphthalene-1,8-dicarboxylic acid, 2-benzoylbenzene-1,3-dicarboxylic acid, 1,3-dibenzyl-2-oxoimidazolidene-4,5-cis-dicarboxylic acid, 2,2'-biquinoline-4,4'-dicarboxylic acid, pyridine-3,4-dicarboxylic acid, 3,6,9-trioxaundecanedicarboxylic acid, hydroxybenzophenone dicarboxylic acid, PLURIOL E300-dicarboxylic acid, PLURIOL E400-dicarboxylic acid, PLURIOL E600-dicarboxylic acid, pyrazole-3,4-dicarboxylic acid, 2,3-pyrazinedicarboxylic acid, 5,6-dimethyl-2,3-pyrazinedicarboxylic acid, 4,4'-diamino(diphenyl ether) diimidodicarboxylic acid, 4,4'-diaminodiphenylmethanediimidodicarboxylic acid, 4,4'-diamino(diphenylsulfone)diimidodicarboxylic acid, 1,4-naphthalene dicarboxylic acid, 2,6-naphthalene dicarboxylic acid, 1,3-adamantanedicarboxylic acid, 1,8-naphthalene dicarboxylic acid, 2,3-naphthalene dicarboxylic acid, 8-methoxy-2,3-naphthalene dicarboxylic acid, 8-nitro-2,3-naphthalene dicarboxylic acid, 8-sulfo-2,3-naphthalene dicarboxylic acid, anthracene-2,3-dicarboxylic acid, 2',3'-diphenyl-p-terphenyl-4,4"-dicarboxylic acid, (diphenyl ether)-4,4'-dicarboxylic acid, imidazole-4,5-dicarboxylic acid, 4(1H)-oxothiochromene-2,8-dicarboxylic acid, 5-tert-butyl-1,3-benzenedicarboxylic acid, 7,8-quinolinedicarboxylic acid, 4,5-imidazoledicarboxylic acid, 4-cyclohexene-1,2-dicarboxylic acid, hexatriacontanedicarboxylic acid, tetradedocanedicarboxylic acid, 1,7-heptanedicarboxylic acid, 5-hydroxy-1,3-benzenedicarboxylic acid, 2,5-dihydroxy-1,4-dicarboxylic acid, pyrazine-2,3-dicarboxylic acid, furan-2,5-dicarboxylic acid, 1-nonene-6,9-dicarboxylic acid, eicosene dicarboxylic acid, 4,4'-dihydroxydiphenylmethane-3,3'-dicarboxylic acid, 1-amino-4-methyl-9,10-dioxo-9,10-dihydroanthracene-2,3-dicarboxylic acid, 2,5-pyridinedicarboxylic acid, cyclohexene-2,3-dicarboxylic acid, 2,9-dichlorofluorobin-4,11-dicarboxylic acid, 7-chloro-3-methylquinoline-6,8-dicarboxylic acid, 2,4-dichlorobenzophenone-2',5'-dicarboxylic acid, 1,3-benzenedicarboxylic acid, 2,6-pyridinedicarboxylic acid, 1-methylpyrrole-3,4-dicarboxylic acid, 1-benzyl-1H-pyrrole-3,4-dicarboxylic acid, anthraquinone-1,5-dicarboxylic acid, 3,5-pyrazoledicarboxylic acid, 2-nitrobenzene-1,4-dicarboxylic acid, heptane-1,7-dicarboxylic acid, cyclobutane-1,1-dicarboxylic acid, 1,14-tetradedocanedicarboxylic acid, 5,6-dehydronorbornane-2,3-dicarboxylic acid, 5-ethyl-2,3-pyridinedicarboxylic acid or camphordicarboxylic acid.

Examples of the tricarboxylic acid comprise 2-hydroxy-1,2,3-propanetricarboxylic acid, 7-chloro-2,3,8-quinolinetricarboxylic acid, 1,2,3-, 1,2,4-benzenetricarboxylic acid, 1,2,4-butanetricarboxylic acid, 2-phosphono-1,2,4-butanetricarboxylic acid, 1,3,5-benzenetricarboxylic acid, 4,4',4"-(1,3,5-benzenetriyl)trisbenzoic acid, 1-hydroxy-1,2,3-propanetricarboxylic acid, 4,5-dihydro-4,5-dioxo-1H-pyrrolo[2,3-F]quinoline-2,7,9-tricarboxylic acid, 5-acetyl-3-amino-6-methylbenzene-1,2,4-tricarboxylic acid, 3-amino-5-benzoyl-6-methylbenzene-1,2,4-tricarboxylic acid, 1,2,3-propane tricarboxylic acid or aurintricarboxylic acid.

Examples of the tetracarboxylic acid comprise perylenetetracarboxylic acids such as 1,1-dioxideperylo[1,12-BCD]thiophene-3,4,9,10-tetracarboxylic acid, perylene-3,4,9,10-tetracarboxylic acid or (perylene-1,12-sulfone)-3,4,9,10-tetracarboxylic acid, butanetetracarboxylic acids such as 1,2,3,4-butanetetracarboxylic acid or meso-1,2,3,4-butanetetracarboxylic acid, decane-2,4,6,8-tetracarboxylic acid, 1,4,7,10,13,16-hexaoxacyclooctadecane-2,3,11,13-tetracarboxylic acid, 1,2,4,5-benzenetetracarboxylic acid, 1,2,11,12-dodecanetetracarboxylic acid, 1,2,5,6-hexanetetracarboxylic acid, 1,2,7,8-octanetetracarboxylic acid, 1,4,5,8-naphthalenetetracarboxylic acid, 1,2,9,10-decanetetracarboxylic acid, benzophenonetetracarboxylic acid, 3,3',4,4'-benzophenonetetracarboxylic acid, tetrahydrofurantetracarboxylic acid and cyclopentanetetracarboxylic acids such as cyclopentane-1,2,3,4-tetracarboxylic acid.

In one embodiment, the organic ligand derives from a heterocycle capable of forming a coordinate bond at the ring heteroatom. Examples of such a heterocycle comprise the following heterocycles. The heterocycle may be unsubstituted or substituted.

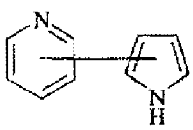
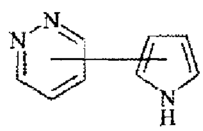
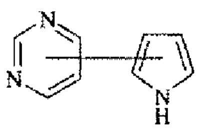
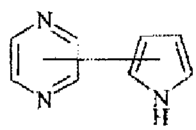

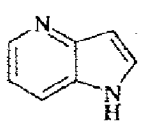
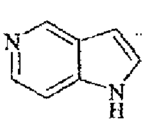
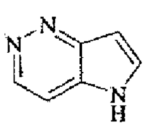
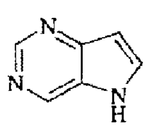
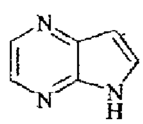

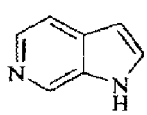
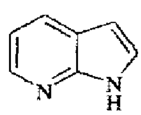
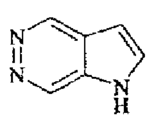
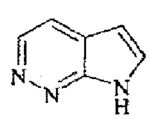
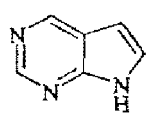

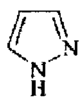 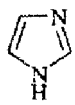 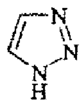 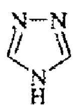 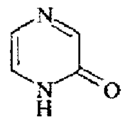 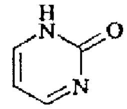 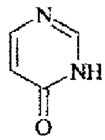

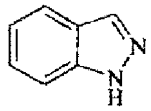 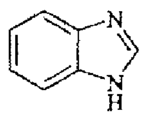 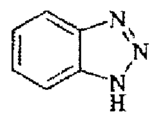 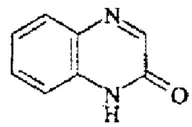

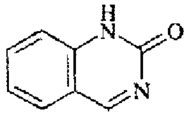 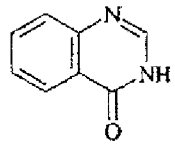

In one embodiment, the organic ligand derives from an azole such as a diazole or a triazole and preferably derived from a triazole.

In a preferable embodiment, the organic ligand comprises an organic ligand that derives from dicarboxylic acid or an organic ligand that derives from an azole, and preferably comprises an organic ligand derived from oxalic acid or an organic ligand that derives from 1,2,4-triazole.

In a preferable embodiment, the organic ligand is selected from 1,4-benzenedicarboxylic acid, 1,2-benzenedicarboxylic acid, maleic acid, 1,3,5-benzenetricarboxylic acid, 4,4',4"-(1,3,5-benzentriyl)trisbenzoic acid, oxalic acid, 4,4'-bipyridine, triazole, imidazole, 3,3'-bipyrazole, benzimidazole or 3,5-pyridinedicarboxylic acid.

The number of types of the organic ligands may be only one, or two or more.

In the present disclosure, "deriving from a compound" may encompass not only the compound itself but also a partially protonated form of such a compound and a fully protonated form of such a compound.

The metal-organic framework can be produced by bringing the metal ion and the organic ligand into contact with each other. Such contact between the metal ion and the organic ligand may be performed in the presence of a solvent. The solvent can be water, ethanol, dimethylformamide, toluene, methanol, chlorobenzene, diethylformamide, dimethylsulfoxide, hydrogen peroxide, methylamine, a sodium hydroxide solution, N-methylpyrrolidone ether, acetonitrile, benzyl chloride, triethylamine, ethylene glycol or a mixture thereof.

The contact between the metal ion and the organic ligand may be performed under increased pressure or under normal pressure. The temperature at the time of performing such contact may be, for example, 10°C to 200°C or 10°C to 150°C. During such contact, stirring may be performed.

The metal ion and the organic ligand may comprise $Zn^{2+}$ and combination of oxalic acid and 1,2,4-triazole, respectively. In such a case, the metal ion can be brought into contact with the organic ligand in a mixture of water and ethanol as the solvent while stirring under normal pressure at a temperature of, for example, 10°C to 30°C, to thereby obtain the metal-organic framework, which provides favorable productivity.

The content of the porous body in the adsorbent is, for example, 50 % by mass or more and 100 % by mass or less, preferably 70 % by mass or more and 100 % by mass or less and more preferably 90 % by mass or more and 100 % by mass or less.

The absorbent may comprise a resin in addition to the porous body. Examples of such a resin comprise an acrylic resin, a polyurethane resin, a polyolefin resin, a polyester resin, a polyamide resin, a vinyl chloride resin, a styrene resin, a vinyl ether resin, a polyvinyl alcohol resin, a polycarbonate resin, or a polysulfone resin.

The absorbent may comprise an additive such as an emulsifier, an antifoaming agent, a surfactant, a leveling agent, a thickener, a viscoelastic modifier, an antifoaming agent, a wetting agent, a dispersant, a preservative, a plasticizer, a penetrant, a fragrance, a bactericide, an acaricide, a fungicide, an ultraviolet absorber, an antioxidant, an antistatic agent, a flame retarder, a dye or a pigment. The adsorbent may be in the form of powder, granules, flakes, or pellets.

(Contact between mixture and adsorbent)

When the mixture and the adsorbent are brought into contact with each other, the fluorocarbon having n carbon atoms in the mixture is adsorbed to the adsorbent, so that the fluorocarbon having n carbon atoms can be separated from the mixture. In one embodiment, the mixture is preferably in the form of a gas when the mixture and the adsorbent are

brought into contact with each other.

The contact between the mixture and the adsorbent may be performed by, for example, a batch method or a column method. The batch method is a method in which the adsorbent and the mixture are put into a sealable container and left still for a certain period of time under conditions of a predetermined temperature and a predetermined pressure, thereby bringing the mixture and the adsorbent into contact with each other. The column method is a method in which a container (column) is packed with the adsorbent and the mixture is made to flow into the container (column), thereby bringing the mixture and the adsorbent into contact with each other.

In one embodiment, the temperature at the time of the contact may be, for example, 10°C or higher and 40°C or lower and, furthermore, 15°C or higher and 35°C or lower. In another embodiment, the temperature at the time of the contact may be, for example, -10°C or higher and 30°C or lower, 0°C or higher and 30°C or lower or 15°C or higher and 30°C or lower. The pressure (gauge pressure) at the time of the contact may be 0.2 MPaG or higher and 2 MPaG or lower or 0.4 MPaG or higher and 1.5 MPaG or lower. When the pressure at the time of the contact is within the above-described range, it is easy for the adsorbate to be adsorbed to the adsorptive medium, so that the separation efficiency can improve, and the pore structure of the adsorptive medium can also be maintained.

In the case of performing the contact by the column method, the flow rate of the mixture may be 50 mL/minute or faster and 1,000 mL/minute or slower or may be 100 mL/minute or faster and 500 mL/minute or slower.

The fluorocarbon having n carbon atoms adsorbed to the adsorbent can be desorbed from the adsorbent by, for example, depressurization or heating. This recycles the adsorbent and makes it possible to collect the fluorocarbon having n carbon atoms.

(Purification)

The separation method of the present disclosure may further comprise purifying the separated fluorocarbon having n carbon atoms. This makes it possible to reuse the fluorocarbon having n carbon atoms.

In one embodiment, the purification can be performed by distillation or rectification. Such distillation may be performed using a generally used distillation tower, for example, a packed tower or a tray tower. A purified product is distilled as a distillate from the top portion of the distillation tower, and the residue is distilled as a bottom from the bottom portion of the distillation tower. The number of theoretical plates of the distillation tower may be, for example, 1 to 100 plates. The pressure (gauge pressure) at the time of the distillation or rectification may be, 0 MPaG or higher and 5 MPaG or lower. The temperature of the top portion of the distillation tower may be, for example, -60°C or higher and 100°C or lower, and the temperature of the bottom portion of the distillation tower may be, for example, 50°C or higher and 200°C or lower. During the distillation, an extraction solvent may coexist.

The rectification can be performed by performing an operation of distilling the distillate again (operation of performing distillation twice or more).

(Composite material)

A composite material comprising an adsorbent and a fluorocarbon having n carbon atoms is also comprised in the technical scope of the present disclosure. In such a composite material, any of the adsorbents described above can be used as the adsorbent, and a metal-organic framework is preferably comprised. A metal ion in the metal-organic framework preferably comprises zinc, and an organic ligand preferably comprises oxalic acid and 1,2,4-triazole.

In the composite material, the content of the fluorocarbon having n carbon atoms is, for example, 1 part by mass or more and 100 parts by mass or less and preferably 10 parts by mass or more and 50 parts by mass or less, per 100 parts by mass of the adsorbent.

In one embodiment, the separation method of the present disclosure can comprise separating a fluorocarbon having a specific molecular diameter from a mixture comprising two or more fluorocarbons having molecular diameters different from each other, and the fluorocarbon having a specific molecular diameter can be separated by bringing the mixture and the adsorbent into contact with each other.

In a preferable embodiment, the separation method of the present disclosure can comprise separating a fluorocarbon having a specific molecular diameter from a mixture comprising the fluorocarbon having a specific molecular diameter and a different fluorocarbon, and the fluorocarbon having a specific molecular diameter can be separated by bringing the mixture and the adsorbent into contact with each other.

In one embodiment, such a separation method comprises separating a fluorocarbon having a molecular diameter of 0.30 nm or more and 0.430 nm or less from a mixture comprising the fluorocarbon having a molecular diameter of 0.300 nm or more and 0.430 nm or less and a fluorocarbon having a molecular diameter of more than 0.430 nm, and the fluorocarbon having a molecular diameter of 0.30 nm or more and 0.430 nm or less can be separated by bringing the mixture and the adsorbent into contact with each other.

In another embodiment, such a separation method comprises separating a fluorocarbon having a molecular

diameter of 0.30 nm or more and 0.460 nm or less from a mixture comprising the fluorocarbon having a molecular diameter of 0.300 nm or more and 0.460 nm or less and a fluorocarbon having a molecular diameter of more than 0.460 nm, and the fluorocarbon having a molecular diameter of 0.30 nm or more and 0.460 nm or less can be separated by bringing the mixture and the adsorbent into contact with each other.

In still another embodiment, such a separation method comprises separating a fluorocarbon having a molecular diameter of 0.30 nm or more and 1.0 nm or less from a mixture containing the fluorocarbon having a molecular diameter of 0.300 nm or more and 1.0 nm or less and a fluorocarbon having a molecular diameter of more than 1.0 nm, and the fluorocarbon having a molecular diameter of 0.30 nm or more and 1.0 nm or less can be separated by bringing the mixture and the adsorbent into contact with each other.

The molecular diameter of the fluorocarbon having a specific molecular diameter can be 0.300 nm or more and 0.430 nm or less in one embodiment, can be 0.300 nm or more and 0.460 nm or less in another embodiment, and can be 0.300 nm or more and 1.0 nm or less in still another embodiment.

In the present embodiment, the boiling point of the fluorocarbon having a specific molecular diameter may be, for example, -80°C or higher and -20°C or lower and, furthermore, may be -60°C or higher and -30°C or lower. In one embodiment, the fluorocarbon having a specific molecular diameter is present as a gas in the mixture.

In the present embodiment, the content of the fluorocarbon having a specific molecular diameter in the mixture may be, for example, 0.1 mol% or more and 50 mol% or less and, furthermore, may be 0.5 mol% or more and 20 mol% or less, based on the entire mixture.

In the present embodiment, the boiling point of the different fluorocarbon may be, for example, -80°C or higher and -20°C or lower, furthermore, may be - 60°C or higher and -30°C or lower and particularly, may be -50°C or higher and - 30°C or lower. In one embodiment, the different fluorocarbon is present as a gas in the mixture.

In the present embodiment, examples of the fluorocarbon having a specific molecular diameter and the different fluorocarbon comprise hydrochlorofluorocarbons and hydrofluorocarbons, and one or more fluorocarbons having predetermined molecular diameters can be selected.

Examples of the hydrochlorofluorocarbons comprise chlorodifluoromethane or chlorotrifluoroethane.

Examples of the hydrofluorocarbons comprise difluoromethane; difluoroethane (for example, 1,1-difluoroethane, 1,2-difluoroethane, particularly, 1, 1-difluoroethane), trifluoroethane (for example, 1,1,1-trifluoroethane, 1,1,2-trifluoroethane, particularly, 1,1,1-trifluoroethane), tetrafluoroethane (for example, 1,1,1,2-tetrafluoroethane, 1, 1,2,2-tetrafluoroethane, particularly, 1,1,1,2-tetrafluoroethane), hydrofluorocarbon having 2 carbon atoms such as pentafluoroethane; hydrofluorocarbons having 3 carbon atoms such as tetrafluoropropene, hexafluoropropene, heptafluoropropane and hexafluoropropane.

In such an embodiment, the content of the fluorocarbon having a specific molecular diameter and the different fluorocarbon in the mixture is preferably 90 mol% or more and 100 mol% or less and more preferably 95 mol% or more and 100 mol% or less, based on the entire mixture.

The mixture may comprise a compound other than fluorocarbons. The boiling point of such a compound may be, for example, -100°C or lower or -150°C or lower. In one embodiment, such a compound is comprised as a gas in the mixture. Examples of such a compound comprise nitrogen, oxygen, carbon dioxide, and water.

In the present embodiment, to the adsorbent and the contact between the mixture and the adsorbent, the same description for the above-described embodiment can be applied. In the present embodiment, the above-described purification may be further performed.

The present embodiment further includes a composite material comprising an adsorbent and a fluorocarbon having a specific molecular diameter. In such a composite material, the content of the fluorocarbon having a specific molecular diameter is, for example, 1 part by mass or more and 100 parts by mass or less and preferably 10 parts by mass or more and 50 parts by mass or less, per 100 parts by mass of the adsorbent.

## Examples

The present disclosure will be more specifically described by way of examples below, but the present disclosure is not limited thereto.

### Examples 1 to 3 and Comparative Examples 1 to 3

As adsorbent, a metal-organic framework 1 (CALF-20, manufactured by Atomis Inc.), a metal-organic framework 2 (NEJSAY, manufactured by Atomis Inc.), a metal-organic framework 3 (MIL-120, manufactured by Atomis Inc.), a metal-organic framework 4 (ZIF-8, manufactured by Atomis Inc.), zeolite 1 (MS-4A, manufactured by Union Showa K.K.) and zeolite 2 (MS-13X, manufactured by Union Showa K.K.) in Tables 1 and 3 were used. The adsorbents to be used were vacuum-treated at 110°C for 12 h, as a pretreatment.

(Measurement of specific surface area)

For the metal-organic frameworks (PCP) 1 to 3 after the pretreatment, the specific surface areas were measured. The specific surface area was measured using an automatic specific surface area/pore size distribution analyzer "BELSORP-mini II" manufactured by MicrotracBEL and calculated by a BET method. The results are listed in Table 1.

| | Name | Center metal | BET($m^2/g$) |
|---|---|---|---|
| Example 1 | CALF-20 | Zn | 400 |
| Example 2 | NEJSAY | Cu | 110 |
| Example 3 | MIL-120 | Al | 50 |

(Measurement of adsorption amount)

For the metal-organic frameworks (PCP) 1 to 3 of Examples 1 to 3 after the pretreatment, the adsorption amounts of chlorodifluoromethane (R22) and pentafluoroethane (R125) were measured at 303 K. As an instrument for measuring the adsorption amount, two instruments listed in the following table were used according to the measurement pressure range.

In addition, for the metal-organic frameworks (PCP) 1 to 4 and zeolites 1 and 2 after the pretreatment, the adsorption amounts were measured to obtain adsorption isotherms. The adsorption amounts when obtaining the adsorption isotherms were measured using an automatic specific surface area/pore size distribution analyzer "BELSORP-max" or "BELSORP-HP" manufactured by MicrotracBEL.

| | Name | Measurement pressure range |
|---|---|---|
| Instrument 1 | High-precision gas and vapor adsorption analyzer "BELSORP-max" manufactured by MicrotracBEL | 0.4Pa~100kPa |
| Instrument 2 | High-pressure gas adsorption instrument "BELSORP-HP" manufactured by MicrotracBEL | 1kPa~1MPa |

Figure 1 shows the adsorption isotherms of chlorodifluoromethane (R22), difluoromethane (R32), pentafluoroethane (R125) and 1,1,1-trifluoroethane (R143a) measured at 303 K regarding the metal-organic framework (PCP) 1 (powder) of Example 1. Figure 1 shows that R22 and R32, fluorocarbons having one carbon atom, were adsorbed to the metal-organic framework 1, and that R125 and R143a, fluorocarbons having two carbon atoms, were hardly adsorbed thereto.

Figure 2 shows the adsorption isotherms of R22 measured at 283 K, 293 K, 303 and 313 K regarding the metal-organic framework (PCP) 1 (powder) of Example 1. Figure 2 shows that the adsorption amount was largest at 283 K, and that there were almost no differences between the adsorption amounts at 293 K to 313 K. For the measurement, "BELSORP-max" was used.

Regarding the metal-organic frameworks (PCP) 2 and 3 of Examples 2 and 3, the metal-organic framework (PCP) 4 of Comparative Example 1 and the zeolites 1 and 2 (powder) of Comparative Examples 2 and 3, the adsorption isotherms of R22 and R125 were obtained in the same manner, and the adsorption amounts at which equilibrium was reached are summarized in Table 3. For the measurement, "BELSORP-HP" was used.

| | Name | Adsorption amount of R22 (mmol) | Adsorption amount of R125 (mmol) | Pore diameter (nm) | Opening diameter (nm) |
|---|---|---|---|---|---|
| Example 1 | CALF-20 | 2.15 | 0 | 0.44 | 0.29 |
| Example 2 | NEJSAY | 0.35 | 0 | 0.45 | 0.37 |
| Example 3 | MIL-120 | 0.65 | 0 | 0.44 | 0.37 |
| Comparative example 1 | ZIF-8 | 0.25 | 0.23 | 1.14 | 0.34 |

(continued)

| | Name | Adsorption amount of R22 (mmol) | Adsorption amount of R125 (mmol) | Pore diameter (nm) | Opening diameter (nm) |
|---|---|---|---|---|---|
| Comparative example 2 | MS-4A | 0.23 | 0.15 | 0.4 | - |
| Comparative example 3 | MS-13X | 2.9 | 3.6 | 13 | - |

Figure 3 shows the adsorption isotherms of R22 and R125 measured at 303 K regarding MS-4A of Comparative Example 2 and MS-13X of Comparative Example 3 as the adsorbents (porous bodies). For the measurement, "BELSORP-HP" was used.

(Measurement of adsorption breakthroughs of gas mixtures)

Figure 4 shows adsorption breakthrough curves obtained by circulating a gas mixture of R125/R22 = 98 mol%/2 mol% into an adsorption tower packed with CALF-20 (pellets) of Example 1 at a total pressure of 0.6 MPaG, a temperature of 298 K and a flow rate of 250 mL/min and measuring the concentrations of R22 and R125 at the outlet of the adsorption tower. The adsorption amount of R22 estimated from the adsorption breakthrough curve was 0.223 g/g, which was almost the same value as the equilibrium adsorption amount of the adsorption isotherm. The molecular diameter of R125 was 0.446 nm, and the molecular diameter of R22 was 0.418 nm.

Figure 5 shows adsorption breakthrough curves obtained by circulating a gas mixture of R125/R32 = 92 mol%/8 mol% into an adsorption tower packed with CALF-20 (pellets) of Example 1 at a total pressure of 0.6 MPaG, a temperature of 298 K and a flow rate of 320 mL/min and measuring the concentrations of R32 and R125 at the outlet of the adsorption tower. The adsorption amount of R32 estimated from the adsorption breakthrough curve was 0.129 g/g, which was almost the same value as the equilibrium adsorption amount of the adsorption isotherm. The molecular diameter of R32 was 0.363 nm.

(Durability test of metal-organic framework to repeated adsorption and desorption)

Figure 6 shows adsorption isotherms obtained by repeating the adsorption and desorption of chlorodifluoromethane (R22) at a temperature of 303 K regarding the metal-organic framework (PCP) 1 of Example 1 and measuring the adsorption amount. For the measurement, "BELSORP-HP" was used. The maximum adsorption amount did not decrease even after 300 repetitions, and the durability to repeated adsorption and desorption is assumed to be high.

(Durability test to high-temperature and high-humidity)

Figure 7 shows adsorption isotherms obtained by measuring the adsorption amounts of chlorodifluoromethane (R22) at 303 K regarding the metal-organic framework (PCP) 1 of Example 1 and samples of the metal-organic framework (PCP) 1 of Example 1 stored under conditions of a temperature of 85°C and a humidity of 95%RH for 14 days, 28 days, 42 days or 56 days. For the measurement of the adsorption isotherms, "BELSORP-HP" was used. For the samples after storage at a high temperature and a high humidity, decreases in the adsorption amounts were suppressed even compared with cases where the samples were not stored at a high temperature or a high humidity, and thus, the durability to high-temperature and high-humidity is assumed to be satisfactory.

## Claims

**1.** A method for separating a fluorocarbon, comprising:

separating a fluorocarbon having n carbon atoms from a mixture comprising the fluorocarbon having n carbon atoms and a fluorocarbon having n + m or more carbon atoms,
wherein the fluorocarbon having n carbon atoms is separated by bringing the mixture and an adsorbent into contact with each other, and
wherein n is an integer of 1 or more, and m is an integer of 1 or more.

**2.** The method according to claim 1, wherein the mixture and the adsorbent are brought into contact with each other at a temperature of -10°C or higher and 30°C or lower.

3. The method according to claim 1 or 2, wherein n is an integer of 1 or more and 2 or less.

4. The method according to any one of claims 1 to 3, wherein m is an integer of 1 or more and 3 or less.

5. The method according to any one of claims 1 to 4, wherein the fluorocarbon having n carbon atoms is at least one selected from the group consisting of difluoromethane and difluorochloromethane.

6. The method according to any one of claims 1 to 5, wherein the fluorocarbon having n + m or more carbon atoms comprises a fluorocarbon having 2 or 3 carbon atoms.

7. The method according to any one of claims 1 to 6, wherein the adsorbent comprises a porous body, and the porous body has an effective pore diameter of 0.46 nm or less.

8. The method according to claim 7, wherein the porous body has a metal-organic framework.

9. The method according to claim 8, wherein the metal-organic framework comprises a metal ion and one or more organic ligands, and
the organic ligand comprises two or more groups capable of forming a coordinate bond to the metal ion, per molecule.

10. The method according to claim 8, wherein the metal-organic framework comprises a metal ion.

11. The method according to claim 8, wherein the metal-organic framework comprises one or more organic ligands, and the organic ligand comprises two or more groups capable of forming a coordinate bond to the metal ion, per molecule.

12. The method according to claim 9, wherein the metal ion comprises zinc, and the organic ligands comprise oxalic acid and 1,2,4-triazole.

13. The method according to any one of claims 7 to 12, wherein the adsorbent further comprises a resin.

14. The method according to any one of claims 1 to 13, wherein the adsorbent is in a form of powder, granules, flakes, or pellets.

15. The method according to any one of claims 1 to 14, wherein the mixture is an azeotropic mixture or pseudoazeotropic mixture of fluorocarbons.

16. The method according to any one of claims 1 to 15, further comprising:
purifying the separated fluorocarbon having n carbon atoms.

17. A composite material comprising: a metal-organic framework; and a fluorocarbon having n carbon atoms,

wherein the metal-organic framework comprises a metal ion and one or more organic ligands,
wherein the metal ion comprises zinc, the organic ligands comprise oxalic acid and 1,2,4-triazole, and
wherein n is an integer of 1 or more.

Figure 1

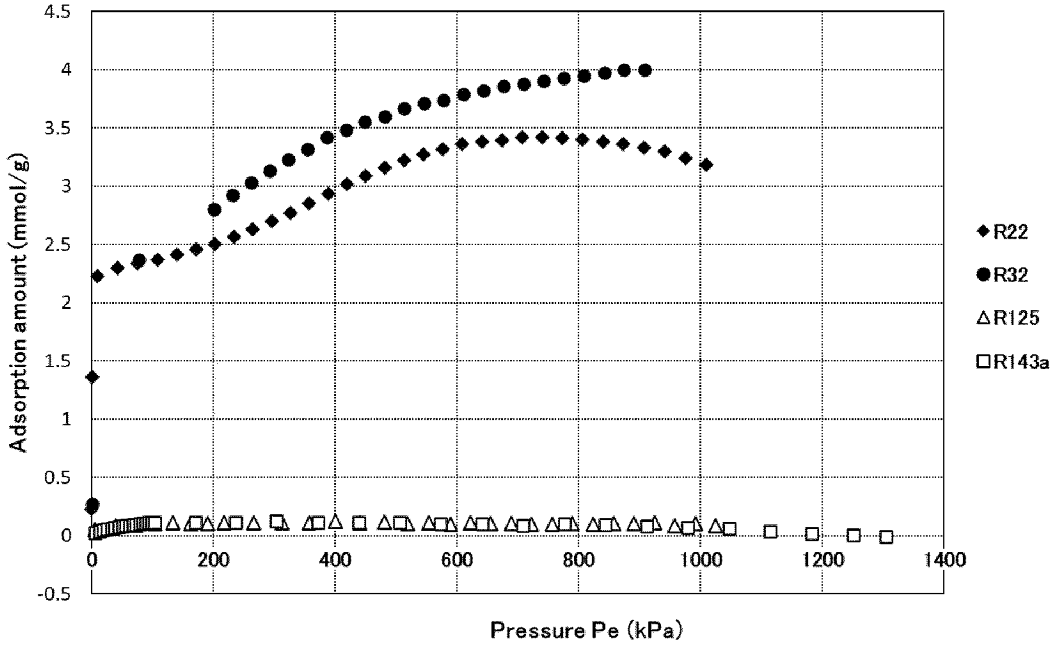


Figure 2

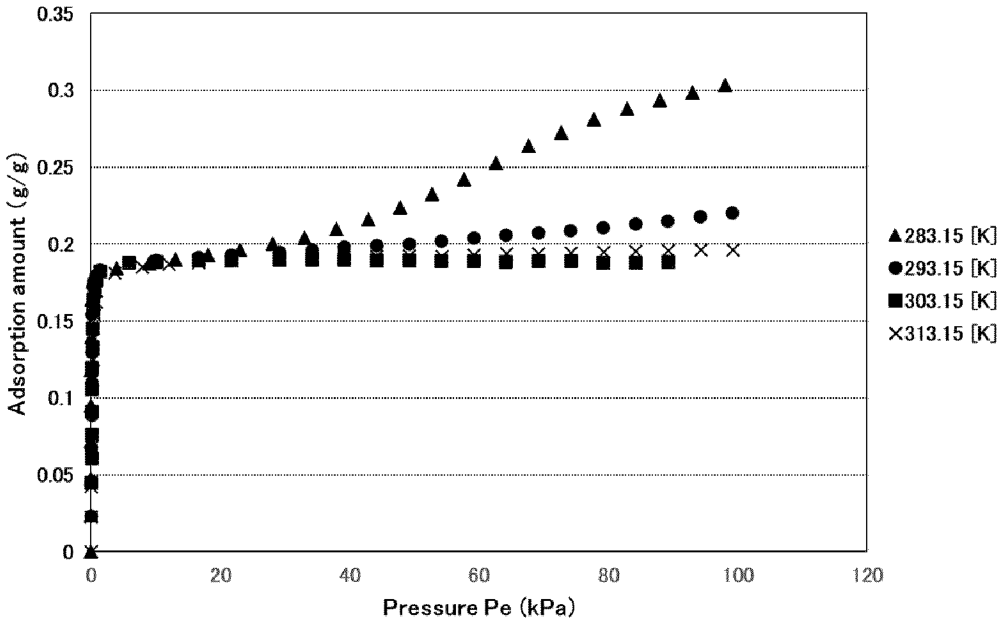

Figure 3

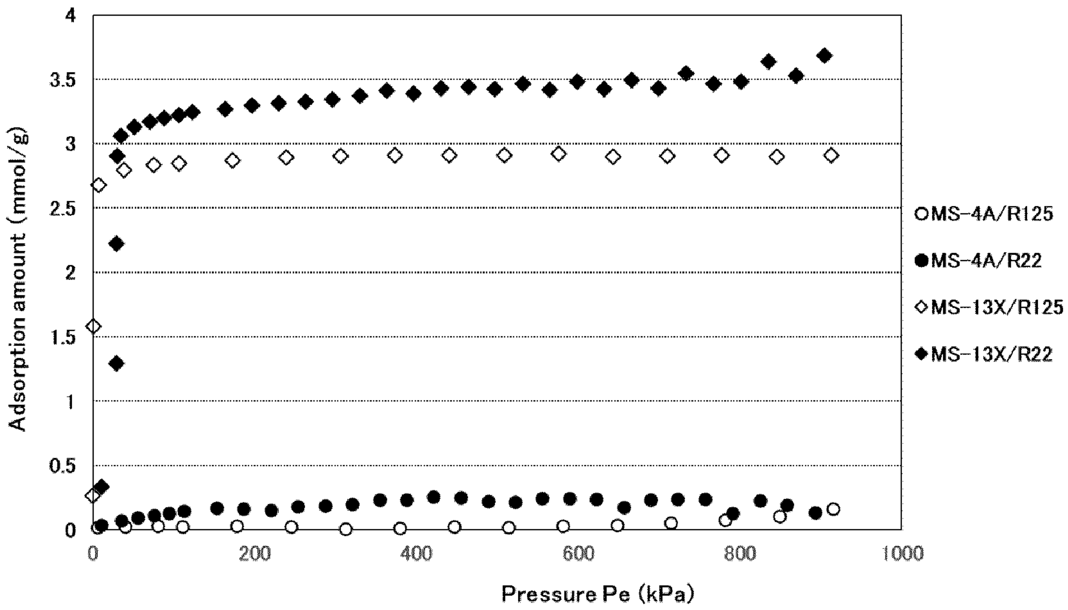


Figure 4

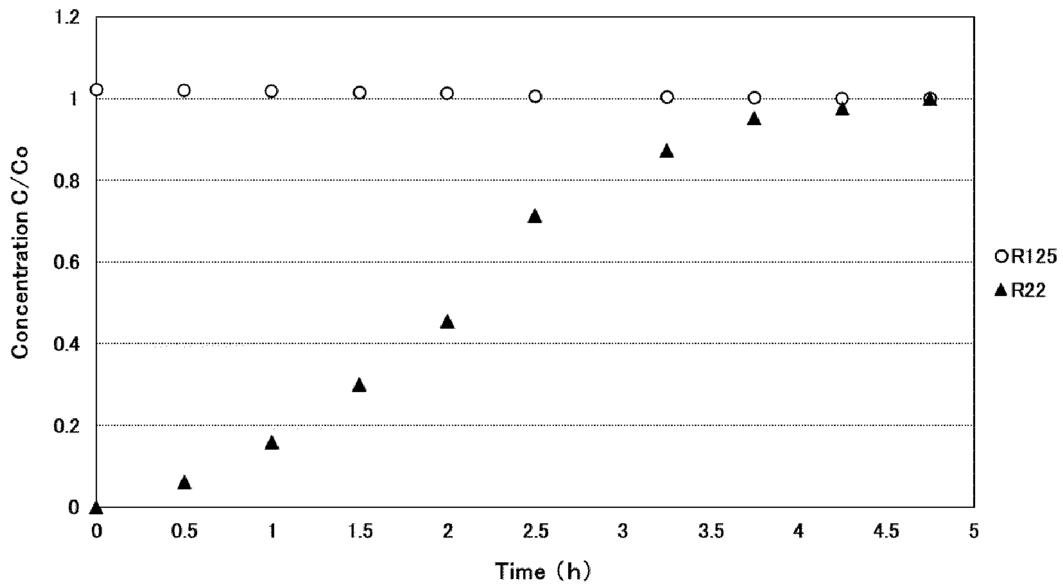

Figure 5

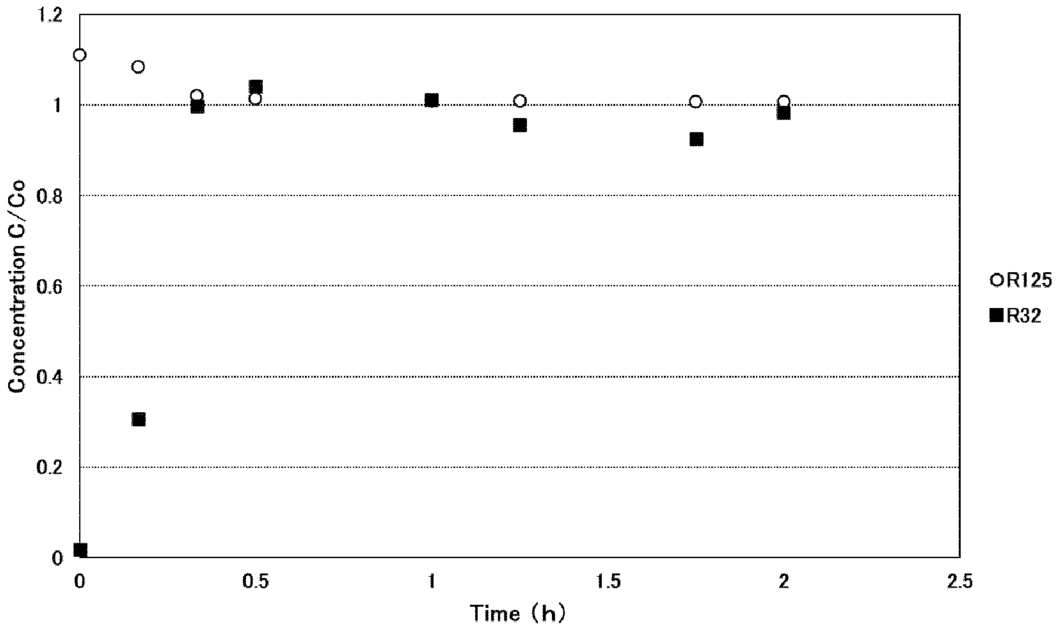


Figure 6

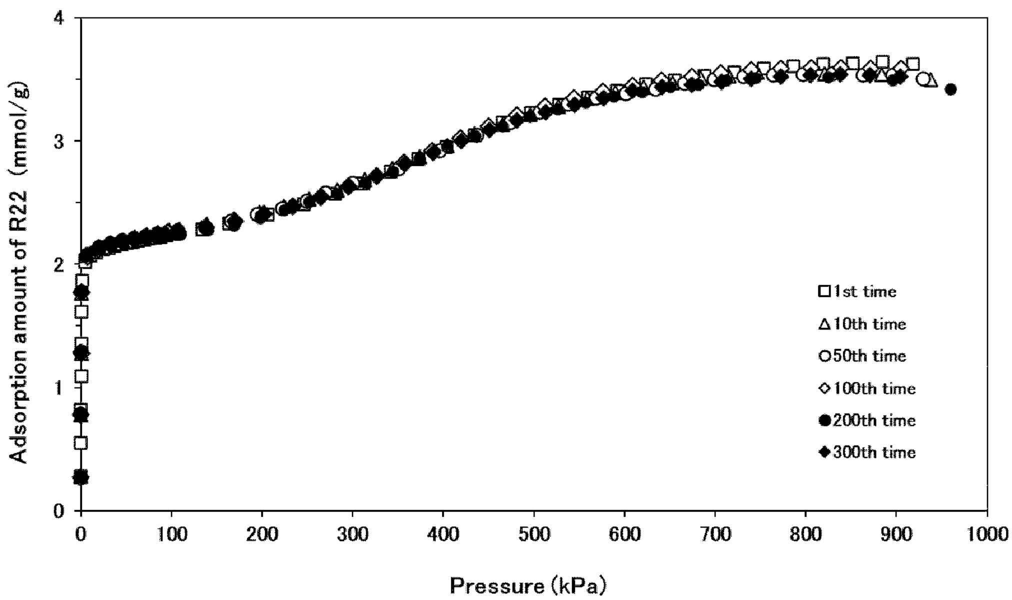

Figure 7

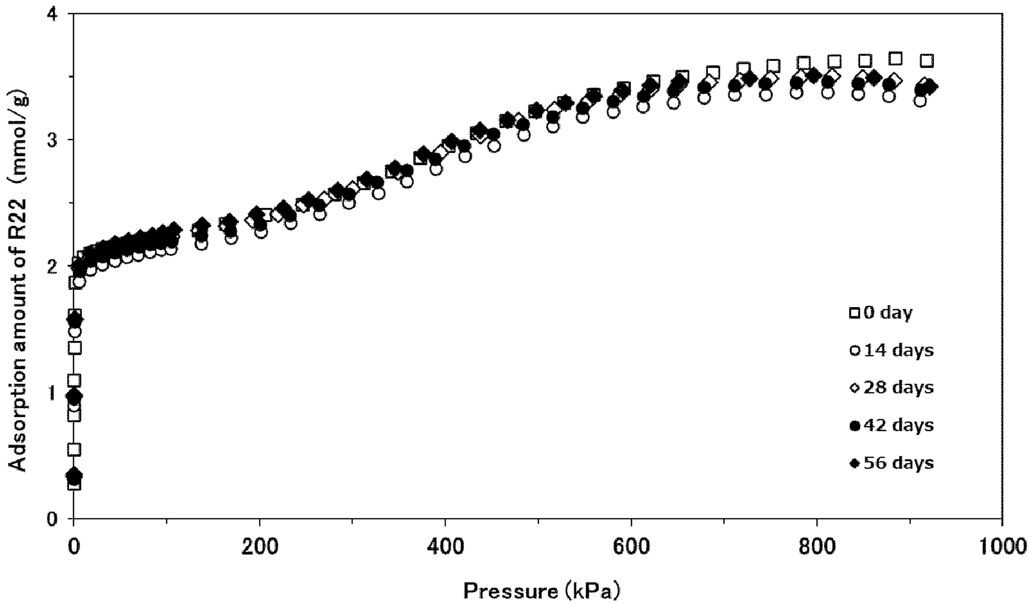
Adsorption amount of R22 (mmol/g)
Pressure (kPa)
0
1
2
3
4
0
200
400
600
800
1000
0 day
14 days
28 days
42 days
56 days

**INTERNATIONAL SEARCH REPORT**

International application No. **PCT/JP2023/006287**

**A. CLASSIFICATION OF SUBJECT MATTER**

***C07C 17/389***(2006.01)i; ***B01D 53/04***(2006.01)i; ***B01J 20/26***(2006.01)i; ***B01J 20/28***(2006.01)i; ***C07C 19/08***(2006.01)i; ***C07C 19/10***(2006.01)i; ***C07C 55/06***(2006.01)i; ***C07F 1/08***(2006.01)i; ***C07F 3/06***(2006.01)i; ***C07F 5/06***(2006.01)i
FI: C07C17/389; C07C19/08; C07C19/10; C07C55/06; C07F3/06; C07F1/08; C07F5/06 D; B01D53/04; B01J20/26 A; B01J20/28

According to International Patent Classification (IPC) or to both national classification and IPC

**B. FIELDS SEARCHED**

Minimum documentation searched (classification system followed by classification symbols)

C07C17/389; B01D53/04; B01J20/26; B01J20/28; C07C19/08; C07C19/10; C07C55/06; C07F1/08; C07F3/06; C07F5/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WANIGARATHNA, Darshika K. J. A. et al. Fluorocarbon Separation in a Thermally Robust Zirconium Carboxylate Metal-Organic Framework. Chemistry - An Asian Journal. 2018, 13(8), 977-981<br>abstract, fig. 2(a), 5(a), 5(c), p. 978, right column to p. 979, left column, p. 980, right column to p. 981, left column, supporting information table S1 | 1-11, 13-16 |
| Y | | 1-11, 13-16 |
| Y | ZHENG, Jian et al. Pore-Engineered Metal-Organic Frameworks with Excellent Adsorption of Water and Fluorocarbon Refrigerant for Cooling Applications. Journal of the American Chemical Society. 2017, 139(31), 10601-10604<br>abstract, fig. 1, 3, etc. | 1-11, 13-16 |
| Y | ZHENG, Jian et al. Molecular Insight into Fluorocarbon Adsorption in Pore Expanded Metal-Organic Framework Analogs. Journal of the American Chemical Society. 2020, 142(6), 3002-3012<br>abstract, fig. 1, 2, etc. | 1-11, 13-16 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 April 2023** | **09 May 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/006287**

**C. DOCUMENTS CONSIDERED TO BE RELEVANT**

| Category* | Citation of document, with indication, where appropriate, of the relevant passages | Relevant to claim No. |
|---|---|---|
| X | WO 2017/029868 A1 (DAIKIN INDUSTRIES, LTD.) 23 February 2017 (2017-02-23) claims, paragraphs [0019], [0026], table 1, fig. 1, etc. | 1-4, 6-11, 13-16 |
| X | JP 2013-241390 A (ASAHI GLASS CO LTD) 05 December 2013 (2013-12-05) claims, paragraphs [0024], [0031], examples | 1-4, 6-7, 13-16 |
| X | JP 2001-302566 A (SHOWA DENKO KK) 31 October 2001 (2001-10-31) claims, paragraph [0022], examples 1-4 | 1-4, 6-7, 13-16 |
| X | KR 10-2021-0129275 A (HONEYWELL INTERNATIONAL INC.) 27 October 2021 (2021-10-27) claims, examples 1-4 | 1-4, 6-7, 13-16 |
| X | JP 2004-307507 A (AIR PRODUCTS & CHEMICALS INC) 04 November 2004 (2004-11-04) claims, example 1, etc. | 1-4, 6, 13-16 |
| X | JP 2004-35436 A (SHOWA DENKO KK) 05 February 2004 (2004-02-05) claims, examples | 1-7, 13-16 |
| A | LIN, Jianbin et al. A scalable metal-organic framework as a durable physisorbent for carbon dioxide capture. Science. 2021, 374(6574), 1464-1469 fig. 1 | 1-17 |
| A | JP 2016-516677 A (UTI LIMITED PARTNERSHIP) 09 June 2016 (2016-06-09) claims, examples, etc. | 1-17 |
| A | VOLKRINGER, Christophe et al. Occurrence of Uncommon Infinite Chains Consisting of Edge-Sharing Octahedra in a Porous Metal Organic Framework-Type Aluminum Pyromellitate Al4(OH)8[C10O8H2] (MIL-120): Synthesis, Structure, and Gas Sorption Properties. Chemistry of Materials. 2009, 21(24), 5783-5791 Experimental Section, figure 1 | 1-17 |
| A | MCGUIRE, Christina V. et al. The surface chemistry of metal-organic frameworks. Chemical Communications. 2015, 51(25), 5199-5217 Introduction, figures (fig. 7, etc.), Conclusion and outlook | 1-17 |

Form PCT/ISA/210 (second sheet) (January 2015)

**INTERNATIONAL SEARCH REPORT**
**Information on patent family members**

International application No.
**PCT/JP2023/006287**

| Patent document cited in search report | Publication date (day/month/year) | Patent family member(s) | Publication date (day/month/year) |
|---|---|---|---|
| WO 2017/029868 A1 | 23 February 2017 | JP 2022-36104 A<br>US 2018/0222823 A1<br>claims, table 1, fig. 1, paragraphs [0014], [0047]<br>US 2020/0239389 A1<br>EP 3339281 A1 | |
| JP 2013-241390 A | 05 December 2013 | (Family: none) | |
| JP 2001-302566 A | 31 October 2001 | US 2003/0034309 A1<br>claims, paragraph [0048], examples 1-4<br>WO 2001/083412 A2<br>CN 1561318 A<br>KR 10-2002-0023966 A | |
| KR 10-2021-0129275 A | 27 October 2021 | JP 2016-511285 A<br>US 2014/0275655 A1<br>claims, examples 1-4<br>JP 2019-48827 A<br>JP 2021-6551 A<br>JP 2022-141845 A<br>US 2017/0001931 A1<br>US 2019/0047927 A1<br>WO 2014/150889 A1<br>EP 2970058 A1<br>EP 3925946 A1<br>KR 10-2015-0131272 A<br>CN 105026351 A | |
| JP 2004-307507 A | 04 November 2004 | US 6669760 B1<br>claims, example 1<br>EP 1466882 A1 | |
| JP 2004-35436 A | 05 February 2004 | US 2005/0124834 A1<br>claims, examples<br>WO 2004/005226 A1<br>CN 1551860 A<br>KR 10-0580915 B1 | |
| JP 2016-516677 A | 09 June 2016 | US 2016/0016148 A1<br>WO 2014/138878 A1<br>EP 2971277 A1<br>CN 105051269 A<br>KR 10-2015-0127218 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018002602 A


### Non-patent literature cited in the description


- *J. Chem. Eng, Jpn.*, 1983, vol. 16 (6), 470-475